Europäisches Patentamt

**European Patent Office**

**Office européen des brevets**

(11) Veröffentlichungsnummer: **0 006 232**
**A1**

(12) # EUROPÄISCHE PATENTANMELDUNG

(21) Anmeldenummer: **79101965.6**

(22) Anmeldetag: **15.06.79**

(51) Int. Cl.³: **A 61 K 7/32**

(30) Priorität: **19.06.78 DE 2826757**

(43) Veröffentlichungstag der Anmeldung: **09.01.80**
Patentblatt 80/1

(84) Benannte Vertragsstaaten: **BE CH FR GB IT NL SE**

(71) Anmelder: **Henkel Kommanditgesellschaft auf Aktien,
Postfach 1100, D-4000 Düsseldorf 1 (DE)**

(72) Erfinder: **Osberghaus, Rainer, Dr., Flösserstrasse 20,
D-4000 Düsseldorf 13 (DE)**

(54) Verwendung einer Kombination von adstringierenden sauren Aluminiumsalzen mit Estern der Citronensäure, Acetylcitronensäure, ein- und zweibasischer aliphatischer Hydroxycarbonsäuren und Antioxidantien als Deodorantien.

(57) Gegenstand der Erfindung ist die Verwendung einer Kombination von adstringierenden sauren Aluminiumsalzen mit Estern der Citronensäure, Acetylcitronensäure, ein- oder zweibasischer aliphatischer Hydroxycarbonsäuren mit 2-4 C-Atomen im Molekül, mit aliphatischen oder alicyclischen Alkoholen mit 1-6 C-Atomen im Molekül und Antioxidantien in kosmetischen Mitteln zur Unterdrückung von Körpergeruch.

EP 0 006 232 A1

4000 Düsseldorf, den 15.6.1978  
Henkelstraße 67

HENKEL KGaA  
ZR-FE/Patente

z-sü

### P a t e n t a n m e l d u n g

### D 5814

"**Verwendung einer Kombination von adstringierenden sauren Aluminiumsalzen mit Estern der Citronensäure, Acetylcitronensäure, ein- und zweibasischer aliphatischer Hydroxycarbonsäuren und Antioxidantien als Deodorantien**"

Adstringierende saure Aluminiumsalze haben sich als brauchbare wirksame Bestandteile von Antitranspirantien erwiesen. Als geeignete saure Aluminiumsalze sind Sulfate, Chloride, Acetate, Borate, Laktate, Sulfamate, Oxyalkylchloride zu nennen, wobei den Aluminiumhydroxychloriden wegen ihrer besonderen Wirkung die größte Bedeutung zukommt. Wenn sich die Aluminiumhydroxychloride auch als sehr gute schweißhemmende Mittel erwiesen haben, so ist ihre Verwendung nicht problemlos, da sie in den wirksamen Konzentrationen zu Unverträglichkeiten Anlaß geben können. Bei einer Konzentrationserniedrigung in den Antitranspirantien läßt dann aber die desodorierende Wirkung oft zu wünschen übrig.

Es wurde nun gefunden, daß sich die desodorierende Wirkung von adstringierenden sauren Aluminiumsalzen, insbesondere von Aluminiumhydroxychloriden erheblich steigern läßt, wenn man sie in Kombination mit Estern /2 der Citronensäure und/oder Acetylcitronensäure, insbesondere deren Triestern, beziehungsweise Estern ein- und zweibasischer aliphatischer Hydroxycarbonsäuren mit 2 - 4 Kohlenstoffatomen im Molekül, insbesondere deren Neutralestern, mit aliphatischen oder alicyclischen Alkoholen mit 1 - 6 Kohlenstoffatomen im Molekül und Antioxidantien verwendet.

Die erfindungsgemäß einzusetzenden Ester der Citronensäure lassen sich in bekannter Weise durch azeotrope Veresterung von Citronensäure mit dem jeweiligen Alkohol herstellen, wie dies z.B. für den Citronensäuretriäthylester in der amerikanischen Patentschrift 2 076 111 (referiert in Chem. Zentralblatt 1937, II., Seite 665) beschrieben ist. Die Herstellung der erfindungsgemäß einzusetzenden Acetylcitronensäureester kann durch Umsetzung der entsprechenden Citronensäureester mit Acetylchlorid, wie dies für den Acetylcitronensäuretriäthylester von Wislicenus in Liebigs Annalen der Chemie, Bd. 129, Seite 192, bzw. durch Umsetzung der entsprechenden Citronensäureester mit Essigsäureanhydrid, wie dies gleichfalls für den Acetylcitronensäuretriäthylester in der amerikanischen Patentschrift 2 445 911 (referiert in Chemical Abstracts 1948, Spalte 8818) beschrieben ist, erfolgen.

Als zu veresternde Alkohole kommen z.B. Methanol, Äthanol, Propanol, Isopropanol, Butanol-1, Butanol-2,2-methylpropanol-1, 2-Methylpropanol-2, 2-Methylbutanol-1, 2-Methylbutanol-4, n-Hexylalkohol, Äthylenglykol, Propylenglykol, Trimethylenglykol, Hexamethylglykol, Glycerin, Erythrit, Sorbit und Cyclohexanol in Frage.

Unter den erfindungsgemäß einzusetzenden Estern der
Citronensäure als auch der Acetylcitronensäure kommt
sowohl von der desodorierenden Wirkung, als auch von
der anwendungstechnischen Eignung den jeweiligen Triestern die größte Bedeutung zu und unter diesen wiederum
den Triestern mit aliphatischen einwertigen Alkoholen
mit 1 - 6 Kohlenstoffatomen im Molekül.

Als erfindungsgemäß einzusetzende Citronensäureester
bzw. Acetylcitronensäureester sind demnach zum Beispiel
Citronensäuremonomethylester, -monoäthylester,
-monopropylester, -monoisopropylester, -mono-n-butylester,
-mono-tert-butylester, -monoamylester, -monohexylester,
-monocyclohexylester, -monoglycerinester, -dimethylester,
-diäthylester, -dipropylester, -diisopropylester,
-dibutylester, -dicyclohexylester, Ester aus 1 Mol
Citronensäure und 2 Mol Propylenglykol, Acetylcitronensäuremonomethylester, -monoäthylester, -monopropylester,
-monobutylester, -monoamylester, -monocyclohexylester,
-dimethylester, -diäthylester, -diisopropylester,
-di-tert.-butylester, -dihexylester, Ester aus 1 Mol
Acetylcitronensäure und 2 Mol Äthylenglykol oder 2 Mol
Hexamethylenglykol, Ester aus 1 Mol Citronensäure
beziehungsweise Acetylcitronensäure und 3 Mol Propylenglykol, insbesondere jedoch Citronensäuretrimethylester,
-triäthylester,-tripropylester, -triisopropylester,
-tri-n-butylester, -tri-tert.-butylester, -triamylester,
-trihexylester, Acetylcitronensäure-trimethylester,
-triäthylester, -tripropylester, -triisopropylester,
-tri-n-butylester, -tri-tert.-butylester, -triamylester,
-trihexylester zu nennen.

/4

0006232

Die erfindungsgemäß einzusetzenden Ester der ein- und zweibasischen aliphatischen 2 - 4 Kohlenstoffatome im Molekül enthaltenden Hydroxycarbonsäuren lassen sich in bekannter Weise durch azeotrope Veresterung der Säuren mit dem jeweiligen Alkohol herstellen. Dabei können als ein- und zweibasische aliphatische Hydroxycarbonsäuren zum Beispiel Glykolsäure, Milchsäure, β-Hydroxypropionsäure, β-Hydroxybuttersäure, Glycerinsäure, Tartronsäure, Apfelsäure, Weinsäure eingesetzt werden.

Als zu veresternde Alkohole kommen z.B. Methanol, Äthanol, Propanol, Isopropanol, Butanol-1, Butanol-2, 2-Methylpropanol-1, 2-Methylpropanol-2, 2-Methylbutanol-1, 2-Methylbutanol-4, n-Hexylalkohol, Äthylenglykol, Propylenglykol, Trimethylenglykol, Hexamethylenglykol, Glycerin, Erythrit, Sorbit und Cyclohexanol in Frage.

Unter den erfindungsgemäß einzusetzenden Estern der aliphatischen Hydroxycarbonsäuren kommt sowohl von der desodorierenden Wirkung als auch von der anwendungstechnischen Eignung den jeweiligen neutralen Estern die größte Bedeutung zu und unter diesen wiederum den neutralen Estern mit aliphatischen einwertigen Alkoholen mit 1 - 6 Kohlenstoffatomen im Molekül.

Als erfindungsgemäß einzusetzende Ester aliphatischer Hydroxycarbonsäuren sind demnach zum Beispiel Glykolsäurecyclohexylester, -glykolester, -glycerinester, Milchsäurepropylenglykolester, -sorbitester, -Oxybuttersäuremethylester, -isopropylester, -hexylester, -erythritester, Glycerinsäurecyclohexylester, -trimethylenglykolester, Tartronsäuremonomethylester, -monoäthylester, -monopropylester, -monobutylester, -monoamylester, -monohexylester,          /5

Tartronsäureglykolester, -glycerinester, Äpfelsäuremonomethylester, -monoäthylester, -monoisopropylester, -monoisobutylester, -mono-tert.-butylester,
-monoamylester, Äpfelsäurecyclohexylester, -propylenglykolester, -hexamethylenglykolester, Weinsäuremonomethylester, -monoäthylester, -monopropylester,
-monoisopropylester, -monobutylester, -monoamylester,
-monohexylester, Weinsäureglykolester, -erythritester,
-sorbitester, insbesondere jedoch Glykolsäuremethylester,
-äthylester, -propylester, -isopropylester, -butylester,
-isobutylester, -tert.-butylester, -amylester, -hexylester, Milchsäuremethylester, -äthylester, -propylester,
-isopropylester, -butylester, -tert.-butylester,
-hexylester, β-Hydroxypropionsäuremethylester, -äthylester, -isopropylester, -butylester, -amylester,
Glycerinsäuremethylester, -äthylester, -propylester,
-butylester, -hexylester, Tartronsäuredimethylester,
-diäthylester, -diisopropylester, -dibutylester,
-diamylester, Äpfelsäuredimethylester, -diäthylester,
-dipropylester, -diisopropylester, -dibutylester,
-dihexylester, Weinsäuredimethylester, -diäthylester,
-dipropylester, -diisopropylester, -dibutylester,
-di-tert.-butylester, -diamylester, -dihexylester zu
nennen.

Als erfindungsgemäß einzusetzende Antioxidantien sind
alle auf dem pharmazeutischen, kosmetischen und Nahrungsmittel-Sektor gebräuchlichen Antioxidantien geeignet
und es sind folgende Produkte zu nennen:
Butylhydroxyanisol, Butylhydroxytoluol, Guajakharz,
Lecithin, Nordihydroguajaretsäure, Propylgallat,
Octylgallat, Dodecylgallat, Tocopherole, Trihydroxybutyrophenon, Ascorbinsäure, Ascorbylpalmitat,
Dilaurylthiodipropionat, Distearylthiodipropionat,
Monoisopropylcitrat, Thiodipropionsäure und Citraconsäure. Besondere Bedeutung kommt dabei dem Butylhydroxyanisol und Butylhydroxytoluol zu. Für den erfindungsgemäßen Einsatz besonders geeignet hat sich ein Gemisch
von 2- und 3-tert.-Butyl-4-hydroxyanisol mit 2,6-Di-
tert.-butyl-4-methylphenol (Butylhydroxytoluol) im
Verhältnis von 1 : 9 bis 9 : 1 erwiesen.

Die erfindungsgemäß zu verwendende Kombination von
adstringierenden sauren Aluminiumsalzen mit Estern
der Citronensäure und/oder Acetylcitronensäure,
beziehungsweise Estern ein- und zweibasischer aliphatischer Hydroxycarbonsäuren mit 2 - 4 Kohlenstoffatomen im Molekül, mit aliphatischen oder alicyclischen
Alkoholen mit 1 - 6 Kohlenstoffatomen im Molekül und
Antioxidantien, kann, sofern es die Zusammensetzung der
Zubereitung im Hinblick auf ihre Verträglichkeit mit den
Aluminiumsalzen zuläßt, in alle üblicherweise für Deodorantien gebräuchliche Zubereitungen eingearbeitet
werden wie Puder, Stifte, Roll-on und Sprays. Die
Einarbeitung erfolgt in bekannter Weise durch einfaches
Vermischen oder Lösen in den anderen Komponenten der
Zubereitung wie Wasser, Lösungsmitteln, Wachsen, Fettsubstanzen, Polyglykolen, Pudergrundstoffen. Die in
den erfindungsgemäßen kosmetischen Mitteln mit desodorierender Wirkung einzuarbeitenden Mengen an adstringierenden sauren Aluminiumsalzen betragen 1 - 20
Gewichtsprozent, vorzugsweise 5 - 15 Gewichtsprozent,
die einzuarbeitenden Mengen an Citronensäureestern
und/oder Acetylcitronensäureestern bzw. Estern ein-
und zweibasischer Hydroxycarbonsäuren mit 2 - 4 Kohlenstoffatomen im Molekül betragen 0,5 - 20 Gewichtsprozent,
vorzugsweise 2 - 10 Gewichtsprozent und die einzuarbeitenden Mengen an Antioxidantien betragen 0,01 - 5
Gewichtsprozent, vorzugsweise 0,1 - 3 Gewichtsprozent,
bezogen auf das gesamte Mittel.

Es ist bereits aus der französischen Patentschrift
2 192 799 bekannt, Antitranspirantien auf Basis von
adstringierenden Salzen zur Vermeidung von Flecken-
und Ränderbildung auf Kleidungsstücken Triäthylester
der Citronensäure oder Acetylcitronensäure zuzusetzen.
Aus diesem Einsatz zur Verhinderung der Fleckenbildung
ließ sich aber in keiner Weise entnehmen, daß durch
eine Kombination mit Estern der Citronensäure, Acetylcitronensäure, ein- und zweibasischer Hydroxycarbonsäuren und Antioxidantien eine erhebliche Steigerung der
desodorierenden Wirkung adstringierender saurer Aluminiumsalze erreicht werden kann.

Die nachfolgenden Beispiele sollen den Gegenstand der
Erfindung näher erläutern, ohne ihn jedoch hierauf zu
beschränken.

/8

## B e i s p i e l e

Für eine vergleichende Wirksamkeitsprüfung wurde ein Panel-Test durchgeführt. Dieser wurde so angelegt, daß in einer Testserie jeweils drei Produkte (A, B und C) vergleichend geprüft werden konnten. Zur Vorbereitung der Probanden wurde an 15 Personen je 1 Stück Seife, die keine antimikrobiellen Wirkstoffe enthielt, mit der Anweisung ausgegeben, während der gesamten Testzeit von 3 Wochen außer dieser Seife weder andere Seifen noch Desodorantien bzw. Antiperspirantien, noch Parfüms zu benutzen. In der ersten Woche, die als Vorbereitungswoche diente, wurde eine unkontrollierte Wäsche mit der vorgeschriebenen Seife durchgeführt. In der darauffolgenden Woche begann der eigentliche Test; Morgens wurde eine von einer Aufsichtsperson kontrollierte Achselwäsche durchgeführt. Die Testprodukte wurden sodann bei je 5 Probanden nach folgendem Schema unter standardisierten Bedingungen auf die linke bzw. rechte Achsel appliziert:

| linke Achsel | rechte Achsel | |
|---|---|---|
| Produkt A | Produkt B | 5 Probanden |
| Produkt A | Produkt C | 5 Probanden |
| Produkt B | Produkt C | 5 Probanden |

In der dritten Woche wurden die Applikationsseiten gewechselt. Nach Applikation der Testprodukte wurden unter den Achseln der Probanden Achselblätter mittels Gummibändern befestigt, die über einen Zeitraum von 7 Stunden getragen werden mußten. Zur Geruchsbeurteilung wurden die Achselblätter nach Abschluß der Testzeit (7 Stunden) abgenommen, in kodierte, verschließbare und auf 37°C erwärmte Glasbehälter gegeben und die verschraubten Gläser anschließend 15 Minuten bei 37°C gelagert. Die Geruchsbeurteilung des links-rechts-Unterschiedes der Achselblätter jeder Versuchsperson (= 1 Prüfobjektpaar) erfolgte durch 3 trainierte Geruchsprüfer, die ihre Bewertung unabhängig voneinander abgaben. Jeder Prüfer erhielt für jeden Testtag ein neues Prüfformular und erstellte die Beurteilung nach folgendem Schema:

| | | |
|---|---|---|
| stärkerer Geruch | = | Note 1 |
| schwächerer Geruch | = | Note 0 |
| gleicher Geruch | ≙ | Note 0,5 für beide Proben. |

Der Testleiter stellte sodann die Summe der Benotungen der Prüfobjektpaare zusammen:

| | $\Sigma$ der Bewertung der Prüfobjektpaare |
|---|---|
| A : B | x Punkte  :  y Punkte |
| A : C | x' Punkte :  y' Punkte |
| B : C | x" Punkte :  y" Punkte |

Durch Addition der Noten, die ein Produkt auf sich vereinigt, erhält man eine Rangfolge, bei welcher die jeweils höhere Gesamtnotensumme die schlechtere Wirkung des Produktes angibt:

| Produkt | Rangfolge |
|---|---|
| A | x + x' Punkte |
| B | y + x" Punkte |
| C | y' + y" Punkte |

Der Montag als jeweils erster Applikationstag der Produkte blieb bei der Auswertung in beiden Testwochen unberücksichtigt.

Aus den nachstehend aufgeführten Tabellen ist die gute
Wirksamkeit der erfindungsgemäßen Kombinationen, die
nicht auf einem rein additiven Effekt beruht, ersichtlich.

In den nachstehenden Versuchen haben die Kurzbezeichnungen die folgende Bedeutung:

BHA        = Stellungsisomerengemisch aus 2- und 3-
             tert.-Butyl-4-hydroxyanisol

BHT        = 2,6-Di-tert-butyl-4-methylphenol

Treibgas 11 = Monofluor-trichlor-methan

Treibgas 12 = Difluor-dichlor-methan

Treibgas 114 = 1,2-Tetrafluordichloräthan.

Bei der beschriebenen Bewertung liegt ein signifikanter
Unterschied auf Basis von mindestens 95 % statistischer
Sicherheit bei einer Punktedifferenz von $\geq$ 19 Punkten
vor.

Versuch 1

Spray A        3,40 % Aluminiumhydroxychlorid
                      $(Al_6(OH)_{15}Cl_3)$
               0,10 % Aerosil
               3,00 % Capryl-/Caprinsäuretriglycerid
              93,50 % Treibgas 11/12 60:40

Spray B        3,40 % Aluminiumhydroxychlorid
               3,00 % Citronensäuretriäthylester
               0,10 % BHA
               0,10 % BHT
              93,40 % Treibgas 11/12 60:40

Spray C        0,16 % 2,4,4'-Trichlor-2'-hydroxy-di-
(Kontrolle)           phenyläther
               2,84 % Capryl-/Caprinsäuretriglycerid
              97,00 % Treibgas 11/12 60:40

| Auswertung | Punkte | Desodorierende Wirkung | Punkte-differenz |
|---|---|---|---|
| Spray A | 119,5 | A > C | 26,5 |
| Spray B | 94,5 | B > A | 25,0 |
| | | B > C | 51,5 |
| Spray C | 146,0 | | |

Versuch 2

Spray A     3,50 % Aluminiumhydroxychlorid
0,10 % Aerosil
3,00 % Citronensäuretriäthylester
ad 100   % Treibgas 11/12 60:40

Spray B     3,50 % Aluminiumhydroxychlorid
0,10 % Aerosil
2,00 % Isopropylmyristat
ad 100   % Treibgas 11/12 60:40

Spray C     3,50 % Aluminiumhydroxychlorid
0,10 % Aerosil
3,00 % Acetylcitronensäuretriäthylester
ad 100   % Treibgas 11/12 60:40

| Auswertung | Punkte | Desodorierende Wirkung | Punkte-differenz |
|---|---|---|---|
| Spray A | 122,5 | A = B | 2,5 |
| | | A = C | 5,0 |
| | | B = C | 2,5 |
| Spray B | 120,0 | | |
| Spray C | 117,5 | | |

Wie den Versuchen in überzeugender Weise zu entnehmen ist, läßt sich eine signifikante Steigerung der desodorierenden Wirkung von adstringierenden sauren Aluminiumsalzen nur durch die erfindungsgemäß zu verwendende Dreierkombination erreichen.

Die erfindungsgemäße Verwendung der Kombination von adstringierenden sauren Aluminiumsalzen mit Estern der Citronensäure und/oder Acetylcitronensäure, bzw. Estern ein- und zweibasischer Hydroxycarbonsäuren mit 2 - 4 Kohlenstoffatomen im Molekül, mit aliphatischen oder alicyclischen Alkoholen mit 1 - 6 Kohlenstoffatomen im Molekül und Antioxidantien kann in kosmetischen Mitteln mit desodorierender Wirkung gemäß nachstehend aufgeführten Rezepturen erfolgen:

Roll-on-Deodorant

| | | |
|---|---|---|
| Aluminiumhydroxychlorid $Al_6(OH)_{15}Cl_3$ (50 %ige Lösung) | 12,0 | Gew.-Teile |
| Wasser | 44,0 | " |
| BHA + BHT 9:1 | 0,2 | " |
| Citronensäuretriäthylester | 3,8 | " |
| Nonylphenolpolyglykoläther | 4,0 | " |
| Äthanol | 15,5 | " |
| Hydroxyäthylcellulose, 5 %ige Lösung | 10,0 | " |
| Sorbitlösung 70 %ig | 10,0 | " |
| Parfüm | 0,5 | " |

Deodorant-Creme

| | |
|---|---|
| Cetyl/Stearylalkohol + ca. 30 Mol Äthylenoxid Eumulgin B 3[R] Dehydag | 12,0 Gew.-Teile |
| Polyol-Fettsäureester, Cetiol HE[R] Dehydag | 20,0 " |
| Citronensäuretriäthylester | 5,0 " |
| BHA + BHT 1:9 | 0,05 " |
| Aluminiumhydroxychlorid $Al_6(OH)_{15}Cl_3$ 50 %ige Lösung | 30,0 " |
| Wasser | 32,0 " |
| p-Hydroxybenzoesäuremethylester | 0,45 " |
| Parfüm | 0,5 " |

Roll-on-Deodorant

| | |
|---|---|
| Aluminiumhydroxychlorid $Al_6(OH)_{15}Cl_3$ (50 %ige Lösung) | 12,0 Gew.-Teile |
| Weinsäurediäthylester | 3,8 " |
| BHA : BHT 1:1 | 0,2 " |
| Nonylphenolpolyglykoläther | 4,0 " |
| Äthanol | 15,5 " |
| Hydroxyäthylcellulose, 5 %ige Lösung | 10,0 " |
| Sorbitlösung 70 %ig | 10,0 " |
| Parfüm | 0,5 " |
| Wasser | 44,0 " |

/14

Patentanmeldung D 5814 = 14 = HENKEL KGaA
ZR-FE/Patente

Deodorant-Creme

Aluminiumhydroxychlorid

$Al_6(OH)_{15}Cl_3$ (50 %ige Lösung)     30,0 Gew.-Teile

Äpfelsäurediäthylester     5,0    "

BHA + BHT 1:1     0,1    "

Cetyl/Stearylalkohol + ca.

30 Mol Äthylenoxid Eumulgin

B 3$^{(R)}$ Dehydag     12,0    "

Polyol-Fettsäureester, Cetiol

HE$^{(R)}$ Dehydag     20,0    "

Wasser     32,0    "

p-Hydroxybenzoesäuremethylester     0,4    "

Parfüm     0,5    "

Desodorierender Spray

Aluminiumoxäthylchlorid

$Al_2(OC_2H_5)_5Cl$     6,0 Gew.-Teile

Citronensäuretriäthylester     3,5    "

BHA + BHT 1:1     0,5    "

Isopropylmyristat     5,0    "

Propylenglykol     3,0    "

Äthanol     12,0    "

Isopropanol     9,0    "

Parfüm     1,0    "

Treibgas 12/114 60:40     60,0    "

0006232

## Deodorant-Lotion

| | | |
|---|---|---|
| Polyol-Fettsäureester, Cetiol HE$^{(R)}$ Dehydag | 6,0 | Gew.-Teile |
| Aluminiumhydroxychlorid $Al_6(OH)_{15}Cl_3$ (50 %ige Lösung) | 20,0 | " |
| Acetylcitronensäuretripropylester | 5,0 | " |
| BHA + BHT 1:1 | 0,5 | " |
| Glycerin | 3,0 | " |
| Äthanol 96 %ig | 50,0 | " |
| Parfüm | 0,5 | " |
| Wasser | 15,0 | " |

Patentanmeldung   D 5814            1            HENKEL KGaA
                                                 ZR-FE/Patente

"Verwendung einer Kombination von adstringierenden sauren Aluminiumsalzen mit Estern der Citronensäure, Acetylcitronensäure, ein- und zweibasischer aliphatischer Hydroxycarbonsäuren und Antioxidantien als Deodorantien"

Patentansprüche:

1. Verwendung einer Kombination von adstringierenden sauren Aluminiumsalzen in einer Menge von 1 - 20 Gewichtsprozent, mit Citronensäureestern und/oder Acetylcitronensäureestern, beziehungsweise Estern ein- und zweibasischer Hydroxycarbonsäuren mit 2 - 4 Kohlenstoffatomen im Molekül, mit aliphatischen oder alicyclischen Alkoholen mit 1 - 6 Kohlenstoffatomen im Molekül, in einer Menge von 0,5 - 20 Gewichtsprozent und Antioxidantien in einer Menge von 0,01 - 5 Gewichtsprozent, jeweils bezogen auf die gesamte Zubereitung, in kosmetischen Mitteln zur Unterdrückung von Körpergeruch.

2. Verwendung der Kombination nach Anspruch 1, dadurch gekennzeichnet, daß die sauren Aluminiumsalze in einer Menge von 5 - 15 Gewichtsprozent, die Citronensäureester und/oder Acetylcitronensäureester bzw. Ester ein- und zweibasischer Hydroxycarbonsäuren in einer Menge von 2 -10 Gewichtsprozent und die Antioxidantien in einer Menge von 0,1 - 3 Gewichtsprozent, jeweils bezogen auf die gesamte Zubereitung, eingesetzt werden.

Europäisches Patentamt

**EUROPÄISCHER RECHERCHENBERICHT**

Nummer der Anmeldung

EP 79 10 1965

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 456 639 (H. SCHWARZKOPF) | 1,2 |
| | * Ansprüche; Seite 3, Absatz 2 bis Seite 4, Absatz 1; Seite 5, Absatz 2 bis Seite 6, Absatz 1; Seite 4, Absatz 4 bis Seite 5, Absatz 1; Beispiele * | |
| | -- | |
| A | GB - A - 1 461 991 (COLGATE PALMOLIVE) | 1 |
| | * Ansprüche; Seite 2, Zeile 98 bis Seite 3, Zeile 72; Beispiele * | |
| | -- | |
| A | FR - A - 2 192 798 (GILLETTE) | 1 |
| | * Ansprüche * | |
| | -- | |
| AD | FR - A - 2 192 799 (GILLETTE) | 1 |
| | * Ansprüche * | |
| | -- | |
| A | BE - A - 631 239 (REVLON) | 1 |
| | * Ansprüche; Seite 8, letzter Absatz * | |
| | -- | |
| A | DE - A - 2 358 121 (BEECHAM) | 1 |
| | * Ansprüche; Beispiele; Seite 4, Absatz 2 * | |
| | ---- | |

**KLASSIFIKATION DER ANMELDUNG (Int.Cl.²)**

A 61 K 7/32

**RECHERCHIERTE SACHGEBIETE (Int. Cl.²)**

A 61 K 7/32

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-09-1979 | VANHECKE |

EPA form 1503.1  06.78